# EUROPEAN PATENT SPECIFICATION

(11) **EP 2 157 734 B1**
(45) Date of publication and mention of the grant of the patent: **28.12.2011**
(21) Application number: 09172247.0
(22) Date of filing: 29.10.2003
(51) Int. Cl.: H04L 12/24, G06F 17/50

(54) **Method and apparatus for identifying components of a network having high importance for network integrity**
Verfahren und Vorrichtung zum Identifizieren von komponenten eines Netzwerks mit grosser Wichtigkeit für die Netzwerkintegrität
Procédé et appareil pour identifier des éléments d'un réseau ayant une grande importance pour l'intégrité du réseau

(30) Priority: 29.10.2002 GB 0225109
(43) Date of publication of application: 24.02.2010
(62) Divisional of application: 08167881.5
(73) Proprietor: E-Therapeutics plc, Newcastle Upon Tyne NE2 4LZ (GB)
(72) Inventor: Young, Malcolm Philip, Newcastle upon Tyne, Tyne and Wear NE 99 (GB); Andras, Peter Emil, Newcastle upon Tyne, Tyne and Wear NE 99 (GB); O'Neill, Mark Anthony, Newcastle upon Tyne, Tyne and Wear NE99 (GB)
(74) Representative: Fox, Nicholas Russell Philip

(56) References cited:
- DOGAN A ET AL: "Matching and scheduling algorithms for minimizing execution time and failure probability of applications in heterogeneous computing" IEEE TRANSACTIONS ON PARALLEL AND DISTRIBUTED SYSTEMS, IEEE SERVICE CENTER, LOS ALAMITOS, CA, US, vol. 13, no. 3, 1 March 2002 (2002-03-01), pages 308-323, XP011094313 ISSN: 1045-9219
- ROBERT TARJAN ED - ROBERT TARJAN: "Depth-first search and linear grajh algorithms" SWITCHING AND AUTOMATA THEORY, 1971., 12TH ANNUAL SYMPOSIUM ON, IEEE, PISCATAWAY, NJ, USA, 13 October 1971 (1971-10-13), pages 114-121, XP031288690
- JULIO VILAR: "Minimal Cuts up to Third Order in a Planar Graph" IEEE TRANSACTIONS ON RELIABILITY, IEEE SERVICE CENTER, PISCATAWAY, NJ, US, vol. R-13, no. 3, 1 August 1984 (1984-08-01), pages 250-256, XP011275090 ISSN: 0018-9529
- HELDEN VAN J ET AL: "REPRESENTING AND ANALYSING MOLECULAR AND CELLULAR FUNCTION USING THE COMPUTER" BIOLOGICAL CHEMISTRY, WALTER DE GRUYTER GMBH & CO, BERLIN, DE, vol. 381, no. 9/10, 1 September 2000 (2000-09-01), pages 921-935, XP008032491 ISSN: 1431-6730
- ZIEN A ET AL: "identification of drug target proteins" ERCIM NEWS, ERCIM, PARIS, FR, 1 October 2000 (2000-10-01), pages 1-3, XP002271388 ISSN: 1564-0094

## Description

The present invention relates to methods of analysing networks of interconnected components to identify components of a network which are of high importance for maintaining the network's integrity. The invention also relates to apparatus for carrying out such methods.

Many sorts of systems can be represented in the form of networks comprising nodes interconnected by links. Examples of such networks are social interactions where the nodes might be individuals and the links interactions between those individuals, the Internet where nodes are computers and the links are communication links between computers, and proteome data where nodes indicate proteins and links indicate exchanges of metabolites or interactions between the proteins.

It has been found that in complex systems often a relatively small proportion of the components in a complex system are vital to its function. Thus for example most single protein species in an intra cellular metabolic network can be removed without affecting the function of the system, as can individual exchanges in a telecommunications network. The reason for this is that there are frequently many alternative routes around any removed or dysfunctional element in a complex system, which alternative routes can yield the same metabolic, physical or informational result.

It is therefore desirable to provide a computer system which can analyse data representative of a network to identify those components which are of high importance for network integrity. In the case of a communications network, if such components can be identified, additional backup can be built to protect the functioning of the vital nodes. In the case of network data representing the proteome of a living organism, the identification of important elements in a network representing the proteome enables potential targets for drug intervention to be identified.

Helden Van J et al : "Representing and Analysing Molecular and Cellular Functions using the computer" Biological Chemistry, Walter de Gruyter GmbH & Co. Berlin, vol. 381, no. 9/10, 1 September 2000 pages 921-935 discusses available databases of proteome data and the manner in which such proteome data can be visualised and processed.

In accordance with one aspect of the present invention there is provided a computer implemented method of identifying target proteins for targeting by drug therapies comprising: obtaining proteome data for an organism to be targeted; storing said proteome data as network data within a computer said network data comprising data defining a plurality of nodes and a plurality of links between said nodes, wherein said nodes are associated with proteins and said links are indicative of protein interactions; processing the stored network data utilizing the computer to determine for each node in the network defined by the stored network data the number of links associated with each node; ordering the nodes in the network defined by stored network data on the basis of the number of links associated with a node; identifying as hub nodes, a set of nodes in the ordered list associated with the greatest numbers of links; and identifying as proteins for targeting by drug therapies, proteins associated with the identified hub nodes.

In accordance with another aspect of the present invention there is provided an information processing apparatus for identifying target proteins for targeting by drug therapies comprising: a data store configured to store proteome data for an organism to be targeted as network data defining a plurality of nodes and a plurality of links between said nodes wherein said nodes are associated with proteins and said links are indicative of protein interactions; a processing unit operable to process network data stored in said data store to determine for each of the nodes of the network defined by the stored network data, the number of links associated with each node; order the nodes in the network defined by stored network data on the basis of the number of links associated with each node; and identify as hub nodes, a set of nodes in the ordered list associated with the greatest numbers of links; and an output unit operable to output data identifying one or more target proteins for targeting by drug therapies proteins corresponding to the identified hub nodes.

Further aspects and embodiments of the present invention will become apparent with reference to the accompanying drawings in which:
Figure 1 is a schematic diagram of the processing of data representing nodes interconnected by links by a computer to identify network components of high importance for the integrity of a network;
Figure 2 is a flow diagram of a method of utilising the computer of Figure 1 as part of a system for identifying pharmaceutical compounds and screening those compounds to identify effective drug treatments;
Figure 3 is a schematic block diagram of the computer modules stored within the memory of the computer of Figure 1;
Figure 4 is a flow diagram of the processing of the computer of Figure 1;
Figure 5 is a schematic block diagram of network data stored within the memory of the computer of Figure 1;
Figure 6 is a schematic illustration of a portion of a network identifying a "hub" node;
Figure 7 is a schematic illustration of a portion of a network identifying a low redundancy node in a network;
Figure 8 is a schematic illustration of a portion of network identifying a low redundancy link in a network;
Figure 9 is a schematic illustration of a portion of a network connecting two sub networks;
Figure 10 is a schematic illustration of a portion of a network identifying a second order node; and
Figure 11 is a schematic illustration of a report identifying possible cellular targets for pharmaceutical compounds.

### Overview of System

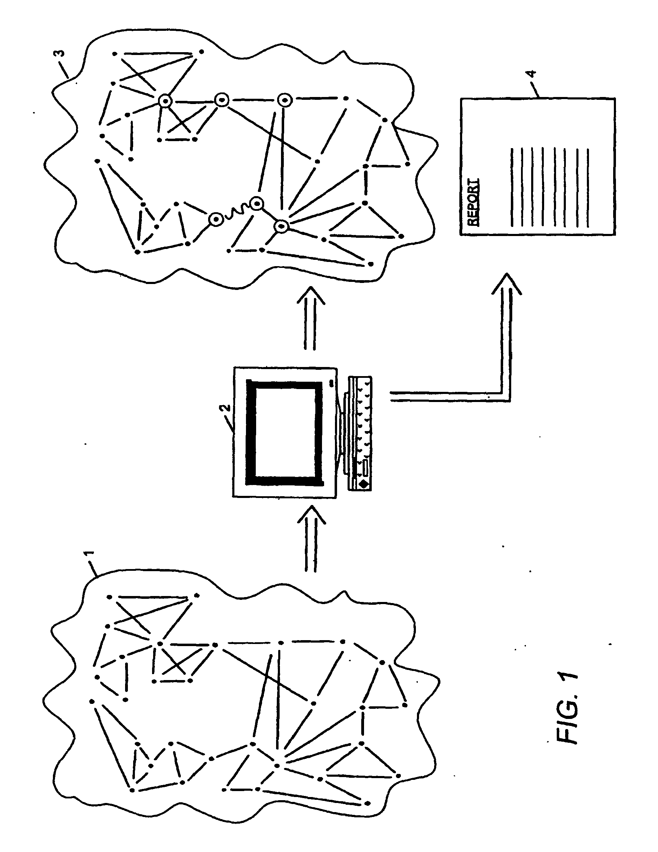

Figure 1 is a schematic illustration of an embodiment of the present invention. In this embodiment data representing a network 1 in the form of nodes (shown as dots in Figure 1) interconnected by links (shown as lines in Figure 1) is input into a computer 2. For illustrative purposes, the data defining a network 1 in this embodiment is taken to be data defining a proteome. That is to say in this embodiment the nodes are indicative of proteins within an organism and the links identify which proteins interact with one another.

Once data representing a network (proteome) has been input into the computer 2, the computer 2 processes the data representing the network to identify within the network a series of nodes and links which are of particular importance for the structural integrity of the network. In Figure 1, processed data is illustrated by network 3 where the identified nodes of importance for structural integrity of the network are illustrated by circles highlighting some of the nodes. In addition, in Figure 1 a link in the network is highlighted as a critical link by a wavy line in the output data 3.

The nodes and links identified as being of importance to the structural integrity of the network 1 by the processing of the computer 2 are established in a number of different ways. Once the critical nodes and links have been identified by the processing of the computer 2, the computer 2 can then output a report 4 identifying the critical nodes and links. In the case of a network 1 representing a proteome of an organism, this report will identify potential drug targets for disrupting the functioning of the organism the proteome 1 represents.

The pharmaceutical industry faces the difficult task of identifying cellular targets for drug intervention. Ordinarily, in a particular cell type, there may be proteome data which identifies between 4000 and 6000 potential proteins which could be possible targets. Checking the effect of disrupting the operation of each protein is therefore very time consuming and expensive, particularly as normally organisms are able to compensate for the disruption of individual proteins.

Previously, heuristic approach and serendipity have been the only means of focussing on potential targets for intervention which are likely to yield biological effects when intervened upon by pharmaceutical chemicals. Targeting multiple proteins in a drug treatment can be more successful. However, the number of potential combinations which could be tested is enormous. Given the costs involved, a more focussed approach is desirable.

The applicants have appreciated that certain topological features of a network enable certain nodes and links to be identified as likely suitable targets since these nodes and links can be identified as being of importance to the structural integrity of a network represented by node and link data. Further, the applicants have determined methods by which groups of target nodes of importance for structural integrity can be identified.

Further, by having the computer 2 store data identifying the critical proteins which are utilised and conserved in a host organism, as will be described the potential targets identified by the computer can be filtered so that the report 4 suggests target proteins of the organism represented by the proteome 1 which are not conserved or utilised by a host organism and hence are more likely not to cause side effects in a host. Additionally, the computer 2 can be arranged to include in the report 4 details of agents which are known to attack the functions of the identified critical proteins.

### Use of System in Treatment Identification

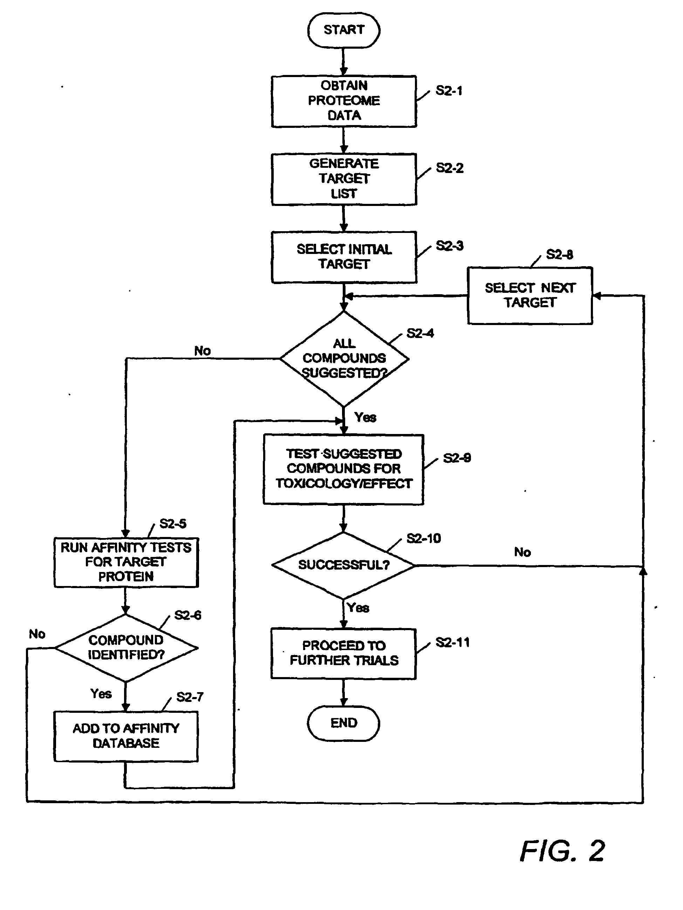

Before describing the structure and functionality of the above computer system 2 in detail, the use of the above system in identifying potential compounds for treating infections will now be described in with reference to Figure 2.

Initially (S2-1) proteome data 1 for a target organism is acquired utilising conventional techniques. This proteome data 1 will identify the proteins present within an organism and also the interactions between those proteins. Identification of the proteins can be achieved using conventional techniques such as mass spectrometry and chromatography etc. Whether different proteins interact can then be established using laboratory techniques such as by manipulating proteins so as to be represented in yeast and seeing whether generated proteins interact. When data for the proteome has been acquired it is then entered into the computer 2 and stored.

The computer 2 then (S2-2) processes the input data in the manner described above so as to generate target data which is output in the form of a report 4. This report will identify lists of potential targets which by virtue of the analysis of the network topology of the stored proteome data 1 will highlight potential targets for intervention.

An initial target identified by the report 4 is then selected (S2-3) and checking the report 4 it is determined (S2-4) whether or not any agents are known to react with the identified protein.

If this is not the case affinity tests can then be run (S2-5) against an expression of the identified protein or proteins to attempt to identify (S2-6) possible compounds that interact with the target. If it is determined that a compound suitable for interacting with the identified target can be found, this data is then added (S2-7) to a compound affinity database for future reference. Alternatively if no such compounds can be found, the next target (S2-8) from the report 4 can be selected for analysis.

Either when one or more compounds for attacking specific targets are suggested by the report 4 or alternatively when suitable compounds have been identified through affinity tests the compound or groups of compounds for targeting the identified protein or proteins can then be tested (S2-9) for toxicology and effect to see whether the combination of compounds does indeed disrupt the activity of the organism represented by the proteome data. If the tests (S2-10) are not successful another set of potential targets from the report 4 can be selected (S2-8) and further potential compounds for therapies can be identified.

If the selected compounds have a desired effect on the organism and are not excessively toxic further trials (S2-11) for the identified compounds can be undertaken to establish whether indeed the identified set of compounds is an effective treatment.

### Structural Components of Computer System

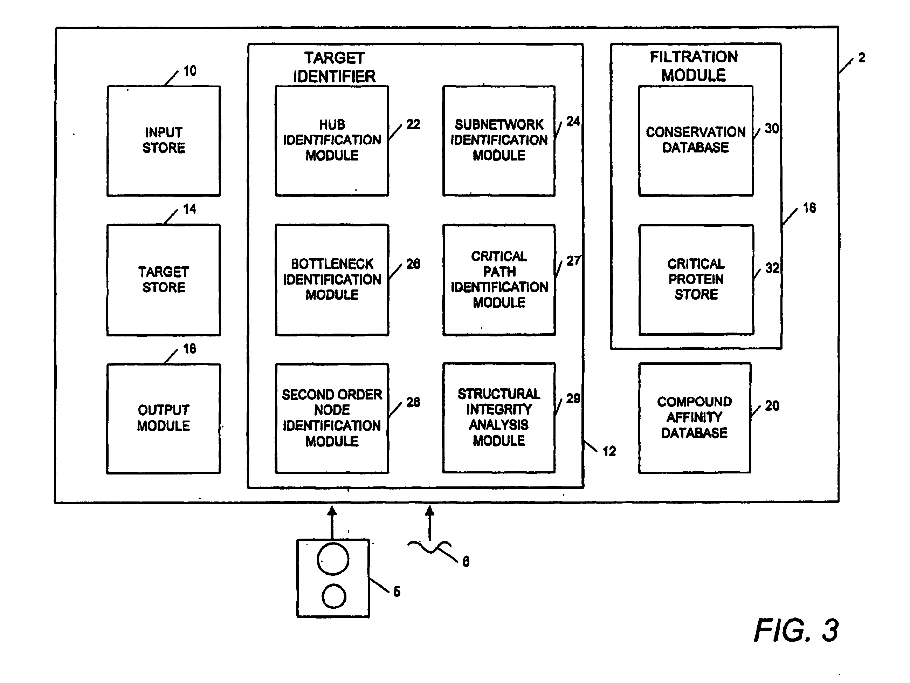

The structure of the computer system of Figure 1 will now be described in detail. Referring to Figure 3 which is a schematic block diagram of the memory of the computer of Figure 1, the computer 2 is programmed to operate in accordance with programming instructions input for example as data stored in a data storage medium such as a disc 5 and/or as a signal 6 input into the computer 2 for example from a remote database by transmission over a communications network (not shown) such as the Internet.

As will be described in more detail below, the programming instructions comprise instructions to cause the memory of the computer 2 to become configured to process input data defining nodes and links in a network. The input data is then processed to generate data identifying critical nodes and links within the network. In the case of input network data defining a proteome where the nodes represent proteins and link interactions between proteins, the identified critical nodes and links will then provide information about potential drug targets.

When programmed by the programming instructions, the memory of the computer 2 effectively becomes configured into a number of functional units for performing processing operations. Examples of such functional units are shown in Figure 3. The units illustrated in Figure 3, are however, notional and are shown for illustration purposes only to assist understanding; they do not necessarily represent exact units and connections into which the processor, memory etc of the computer 2 become configured.

Referring to the functional units shown in Figure 3, an input store 10 is provided for storing data defining network data. In this embodiment which is arranged to process proteome data, this network data comprises data identifying proteins in an organism and known interactions between those proteins.

A target identifier 12 is provided which is arranged to process the network data stored within the input store 10 to identify critical proteins and protein interactions having high importance for the integrity of the proteome. Data identifying the critical proteins is then stored within a target store 14. When the target identifier 12 has stored within the target store 14 data identifying critical proteins, the data within the target store 14 is then filtered utilising a filtration module 16 to identify critical proteins and proteins which are conserved within a host organism.

Finally, an output module 18 utilises the filtered data within the target store 14 and a compound affinity database 20 containing data identifying compounds known to react with proteins to generate and output a report 4 which could be displayed on a screen (not shown) or printed on a printer (not shown) listing the identified critical proteins together with suggested compounds for therapies based on drug targets identified by the target identifier 12.

In this embodiment, the target identifier 12 comprises six sub modules 22-29 each arranged to identify a different type of structure within network data which is indicative of particular components in the network being of high importance for the structural integrity of the network.

The sub modules comprise a hub identification module 22 which is arranged to identify proteins which interact with large numbers of other proteins; a sub network identification module 24 for identifying connections between sub networks; a bottleneck identification module 26 and a critical path identification module 27 for identifying nodes and links within the network data in the input store 10 which cannot be easily bypassed and hence are of importance for the integrity of the network; a second order node identification module 28 for identifying nodes representing proteins directly interacting with nodes identified by the hub identification module 22, sub network identification module 24 and bottleneck identification module 26; and a structural integrity analysis module 29 for identifying groups of nodes which together significantly affect the structural integrity of the network represented by the data within the input store 10.

As will be described after targets and proteins have been identified by the target identifier 12 and stored within the target store 14, the targets are filtered by a filtration module 16. In this embodiment, the filtration module 16 comprises a conservation database 30 and a critical protein store 32.

The conservation database 30 is arranged to store data identifying similar proteins which are conserved between different organisms. Thus for example data is stored identifying that a particular protein in an organism is substantially a homolog of another protein in a host such as a human. The critical protein store 32 is a database storing data identifying critical proteins for the activity of a host. When data identifying a number of target proteins has been generated and stored within the target store 14, the stored targets are likely to be proteins and metabolites which will disrupt the activity of the organism identified by the proteome data and the input store 10 by virtue of the manner of the processing by the target identifier 12. However, although such targets may be useful for enabling general disinfectants to be identified, if a suitable drug is to be developed it must not only be effective against a target organism, but also must not have excessive side effects.

In order to aid with the identification of more promising drug targets, the filtration module 16 stores in the conservation database 30 data for identifying which proteins have similar proteins in the host organism. Where a potential target protein is identified which is not present in any form in a host organism it is more likely that a therapy disrupting that particular protein will have limited side effects. If it is not possible to identify a protein which is not conserved as a potential target, at the very least it is desirable to ensure that the targets chosen for further research are unlikely to disrupt the critical systems of a host. By storing data in a critical protein store 32 identifying the critical proteins for a host the list of potential targets can be appropriately filtered to highlight the most promising potential therapies.

### Processing to Identify Critical Nodes and Links

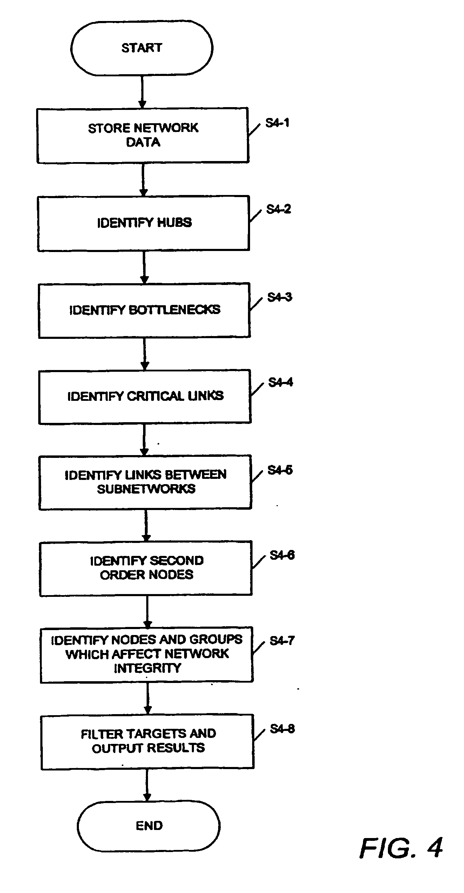

The processing of the computer 2 will now be described in greater detail with reference to Figure 4 which is a flow diagram illustrating the processing of the computer 2.

Initially (S4-1) data representing the network to be analysed is stored within the input store 10.

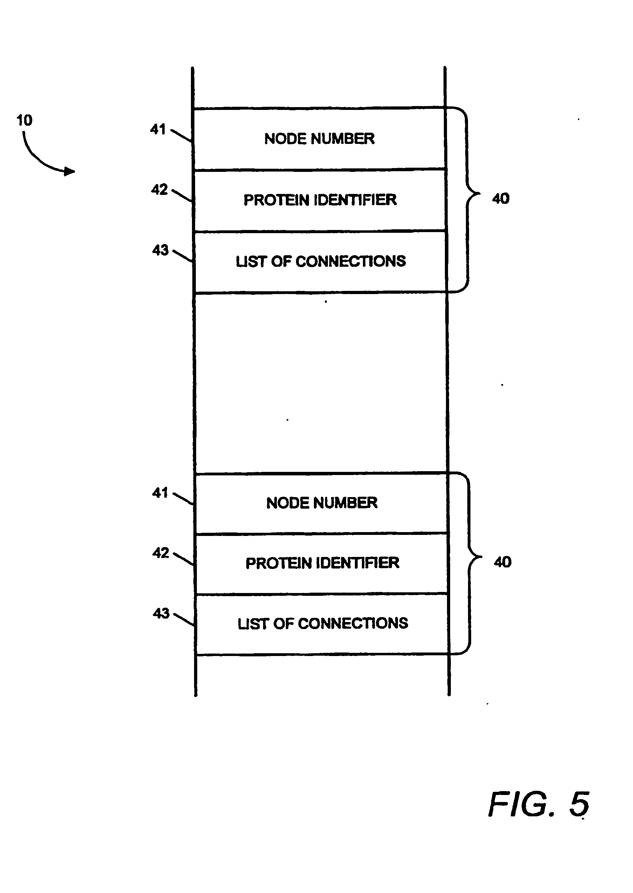

Figure 5 is a schematic illustration of data stored within the input store 10. In this embodiment the data stored within the input store 10 is stored in the form of a number of node records 40 each comprising a node number 41, a protein identifier 42 and a list of connections 43. One of these records 40 is stored for each of the proteins within the proteome being analysed. In each record 40 the list of connections 43 is a list of node numbers 41 of the node records 40 of the proteins with which the protein identified by the protein identifier 42 for the record 40 is known to interact with. Such data can be obtained for a proteome for a particular organism or cell utilising conventional laboratory techniques.

In other embodiments where the network data stored within the input store 10 is representative of a network other than a proteome, the protein identifier 42 will be replaced with a different identifier of a network component and the list of connections 42 would be a list of node numbers 41 of components within the network an identified component interacts with.

### (a) Hub Identification

Once data for the proteome has been stored within the input store 10, the.target identifier 12 then invokes the hub identification module 22 to identify (S4-2) hub nodes within the network.

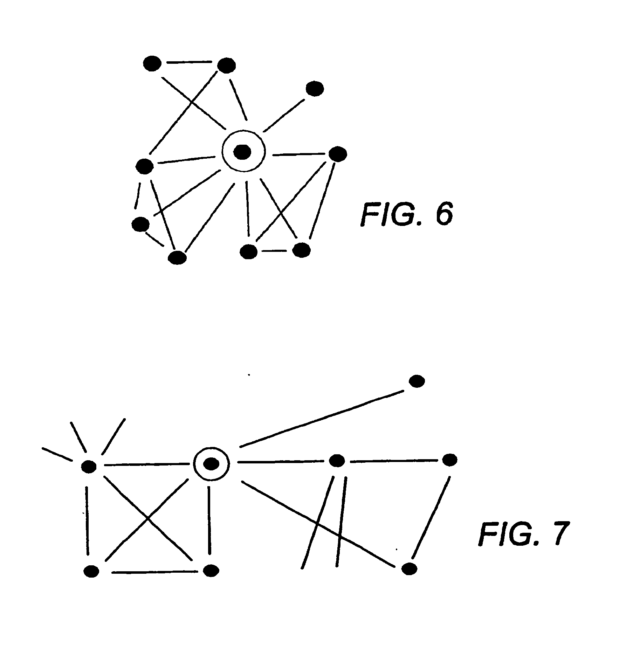

Figure 6 is a schematic illustration of a portion of a network. In Figure 6 nodes are represented by circles and links between nodes are indicated by lines connecting the circles. As shown in Figure 6 some nodes such as the node highlighted by a larger circle interact with significantly more nodes than average. Where nodes represent proteins such well connected nodes are often indicative of proteins critical to the functioning of an organism. By identifying such nodes, potential drug targets can therefore be found.

Thus, in this embodiment, when the hub identification module 22 is invoked, the hub identification module 22 initially determines for each of the node records 40 within the input store 10 the number of entries in the list of connections 43 for each record 40. A list of node numbers is then ordered according to the number of entries in the list of connections 43 in the records 40 identified by the node numbers 41.

Thus in this way the node numbers 41 of nodes which have the greatest numbers of connections and hence are indicative of hubs within the network can be identified. Data identifying the node numbers of nodes with the greatest number of connections is then stored in the target store 14.

In this embodiment which is arranged to process proteome data where normally approximately around about 4000-6000 proteins are included in a proteome and hence 4000-6000 node records 40 will ordinarily be stored in the input store 10. When this number of nodes is stored the hub identification module 22 in this embodiment is arranged to store within the target store 14 the node numbers identifying the twenty nodes having the greatest number of entries in their list of connections 43.

Returning to Figure 4, after storing data indicative of the hub nodes in the target store 14, in this embodiment the target identifier then (s4-3-s4-7) proceeds to identify potential drug targets in a number of other ways which will now be described in outline.

### (b) Bottleneck Identification

Having stored data indicative of the hub nodes in the target store 14, the target identifier 12 then (S4-3) invokes the bottleneck identification module 26 to identify within the network represented by data stored within the input store 10 further portions of a network which are important for the structural integrity of that network.

Specifically, the bottleneck identification module 26 is arranged to identify nodes in the network which cannot be easily bypassed. An example of such a node within a network is illustrated in the exemplary network of Figure 7 where all the paths from the nodes shown as dots in the network of Figure 7 pass through a single node highlighted by a circle. If communication through the node highlighted by the circle is disrupted this then has a significant impact on the integrity of the rest of the network as many nodes will no longer be able to communicate with one another.

### (c) Critical Link Identification

After the bottleneck identification module 26 has identified and stored data identifying any nodes which are difficult to bypass in the network, the critical path identification module 27 is then invoked and attempts to identify (S4-4) individual links within the network which are difficult to bypass.

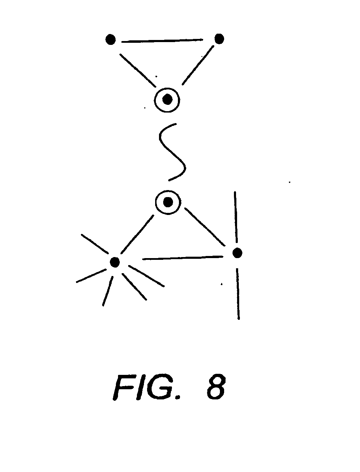

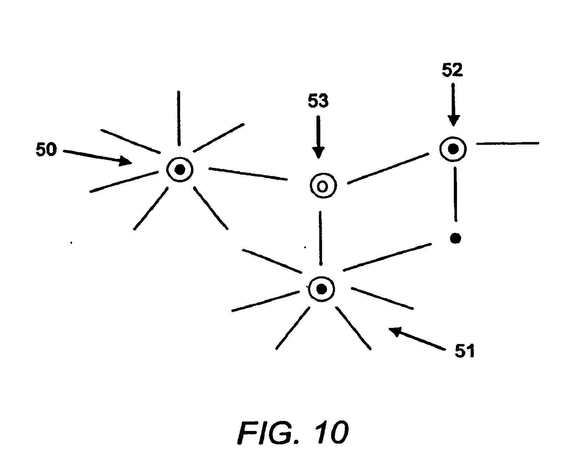

Figure 8 is a schematic illustration of a portion of a network where a critical link between two nodes is highlighted. In the case of Figure 10 the highlighted nodes are surrounded by larger circles and the highlighted link is illustrated by a wavy line.

When processing data to identify nodes that are difficult to bypass such as that illustrated in Figure 8, often these critical nodes will be connected to one another. In such circumstance in addition to identifying the nodes as of importance for the structural integrity of the network, the individual link between two nodes can also be identified as a potential weakness within the network.

### (d) Identification of Links Between Sub Networks

After the critical path identification module 27 has identified links within the network which cannot easily be bypassed, the sub network identification module 24 is then invoked which then proceeds to identify (S4-5) nodes and links involved in connecting sub networks.

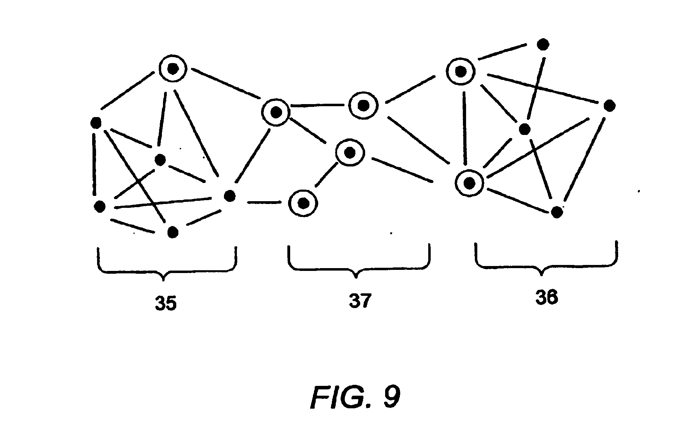

Figure 9 is a schematic illustration of a network divided into two sub networks. In this application the term sub network is taken to mean portions of a network comprising nodes that are more connected to one another than other nodes in the rest of the network. Thus in the case of Figure 9 the left and right hand sections of the illustrated network 35,36 are examples of sub networks whereas the nodes in the centre of the illustrations 37 are an illustrative example of a bridge between two sub networks. That is to say the nodes shown as highlighted provide a connection between the two sub networks 35,36.

When network data is representative of for example a proteome, the existence of sub networks normally identify a series of proteins and protein interactions responsible for different functions within the organism. Thus for example one sub network might involve proteins responsible for controlling cell division, whereas another sub network might identify proteins responsible for controlling energy generation.

By identifying nodes responsible for linking the activities of two sub networks, it is possible to identify targets which disturb communications between the sub networks. In the case of an organism, this could for example cause the functions responsible for cell division to no longer be co-ordinated with the energy generation network and hence cause the organism to no longer reproduce properly.

### (e) Identification of Second Order Nodes

At this stage stored within the target store 14 is data identifying hub nodes, nodes that are difficult to route around and nodes involved in links between sub networks. Each of the sets of nodes will have been identified utilising the node and link data defining a network topology stored in the input store 10.

In addition to these nodes, the applicants have appreciated that a further set of nodes that are important for network integrity are those nodes that are connected to these identified hubs, nodes that are difficult to avoid and links between sub networks. This is because these nodes interact with nodes of importance and hence if the functioning of these connected nodes is disrupted, the functioning of the other identified nodes of importance may also be effected.

In the case of proteome data identifying proteins and protein interactions, frequently, certain proteins corresponding to hubs or other critical nodes are in practice unsuitable for targets as disrupting the activity of such protein can cause unwanted side effects in a host. The secondary proteins which interact with these critical proteins may, however, differ between a target organism and a host. By interfering with the manner in which these proteins interact with the identified critical nodes, the activities of these critical nodes can be effected in a way does not cause a corresponding disruption of the activity of a host.

Thus for example in Figure 10 there are four nodes, two of these nodes labelled 50 and 51 are examples of hub nodes having many connections. Node 52 is shown as an example of a node which is difficult to route around. In the exemplary network of Figure 16, node 53 is shown as being connected to nodes 50, 51 and 52, all of which can be identified as being of potential importance by virtue of analysis of the network topology. Given the large number of links node 53 has to nodes identifiable as important, enables node 53 to be identified as a potential target for effecting the structural integrity of the network.

Thus returning to Figure 4, in this embodiment once the hub nodes, nodes linking sub networks and nodes which are difficult to route around have been identified, the target identifier 12 invokes the second order node identification module 28. The second order node identification module 28 then (S4-6) determines for each of the nodes in the network the number of nodes for which identifying data has been stored in the target store 14 which are contained in the list of connections 43 in each of the node records 40. This data is stored for each of the nodes and the second order node identification module 28 then identifies the top twenty nodes connected to the greatest number of other nodes of importance.
Thus in this way the second order node identification module 28 is able to identify those nodes which are directly linked to a number of other nodes of importance.

### (f) Identification of Groups of Nodes for Affecting Network Integrity

At this stage, the target store 14 will have stored within it data identifying the node numbers of all of the nodes identified by the hub identification module 22, sub network identification module 24, bottleneck identification 26, critical path identification module 27 and second order node identification module 28. Although this data identifies individual nodes of importance for maintaining the structural integrity of the network identified by data stored within the input store 10, it is desirable for the target identifier 14 to additionally generate data identifying groups of nodes which together effect the structural integrity of the network. In this embodiment this is achieved by the structural integrity analysis module 29 which proceeds to identify (S4-7) nodes and groups of nodes which effect network integrity.

At this stage as a result of the processing of the target identifier 12, the target store 14 will have stored data identifying hub nodes, nodes involved in connections between sub networks, nodes which are difficult to route around, links that are difficult to route around or are involved in connections between sub networks and groups of nodes which together significantly effect the structural integrity of the network defined by the network data stored within the input store 10.

### (g) Filter targets and output results

After the processing of the structural integrity analysis module 29 has been completed, the output module 18 is invoked (S4-8) which processes the data stored within the target store 14 utilising the filtration module 16 and the compound affinity database 20 to generate a report 4 as will now be described.

Specifically in this embodiment each of the nodes identified by data within the target store 14 is checked against the conservation database 30 and the critical protein store 32 to determine whether the node number identified by data stored within the target store corresponds to the node number 41 of a node record 40 identifying a protein 42 corresponding to a protein stored within the conservation database 30 or the critical protein store 32.

In this way the output module 28 is able to classify each of the items of data stored within the target store 14 as either relating to critical proteins identified by data within the critical protein store 32, proteins corresponding to proteins identified by the conservation database 30 or neither of these.

The output module 18 then generates and outputs a report 4 which identifies the proteins corresponding to the node numbers stored within the target store 14 where the proteins which are determined not to appear in either of the conservation database 30 or the critical protein store 32 are listed separately from those which are determined to appear in the conservation database 30 or the critical protein store 32.

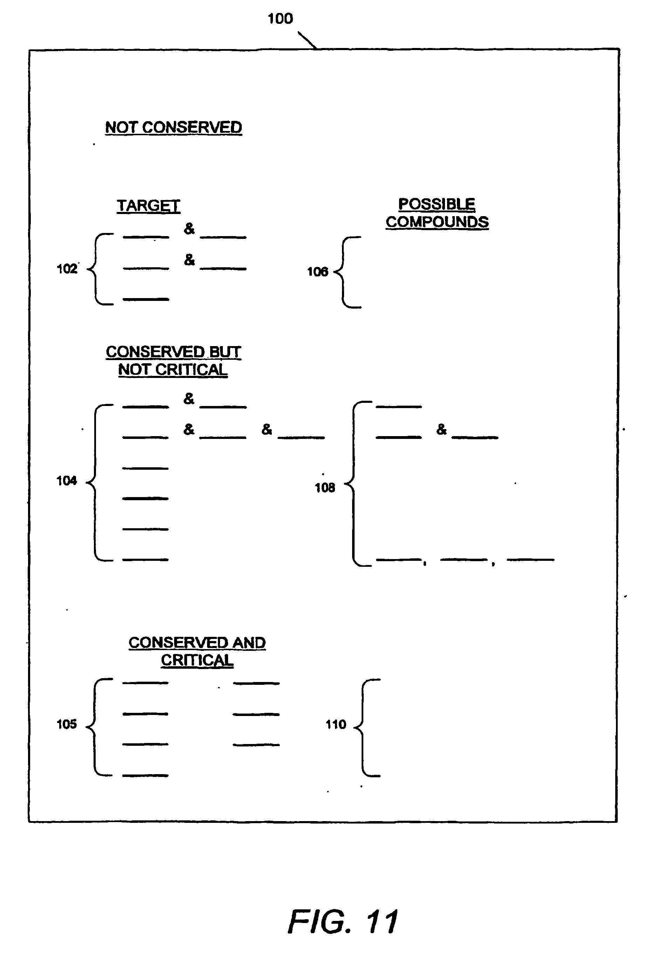

Figure 11 is a schematic illustration of a report 100 generated by the output module 18.

In this embodiment the report 100 comprises three lists 102, 104, 105 where the first list 102 identifies proteins identified by data stored in the target store 14 for which no corresponding entries are stored within the conservation database 30 or critical protein store 32; a second list 104 which identifies proteins identified by data stored within the target store 14 where any of the nodes or nodes within the groups of nodes are identified by data within the conservation database 30 but not the critical protein store 32; and a third list 105 which identifies the remaining proteins identified by data in the target store 14.

In this embodiment adjacent to each of these lists is a further list 106,108,110. Each of these lists identify for the corresponding list within the report 100 any compounds known to react with proteins identified in the list as identified by data within the compound affinity database 20. Thus in this way the output module 18 is able to generate a report where possible target proteins are identified based on an analysis of the topology of network data input into the input store 10.

### Modifications and Amendments

In the above described embodiment, a filtration module 16 is described as including a critical protein store 32 identifying critical proteins for the functioning of a host organism. The data entered into the critical protein store could be obtained through conventional sources. Alternatively the system described could be utilised to identify critical proteins.

Specifically instead of entering proteome data into the input store 10 representative of the proteome of an organism to be attacked, proteome data for the host organism could be entered into the input store. When this host organism proteome data was processed, the target identifier 12 would then proceed to identify hubs, nodes and links involved in connection between sub networks, nodes and links that are difficult to route around and second order nodes and groups of nodes which effect the structure integrity of the network represented in the host organism proteome.

Just as in the case of processing proteome data representative of an organism to be attacked, this processing will identify nodes, links and groups of nodes which are important for the structural integrity of the host organism proteome. By generating data in this way identification of critical proteins, links and groups of protein for a hosting organism could then be achieved.

More generally whenever two networks interact with one another by processing data representative of a first network and storing data identifying critical elements in that first network and then processing data for the second network, it is possible to identify critical elements in the second network, interference with which is less likely to effect the functioning of the first network.

Although in the above described embodiment, the input of data corresponding to a proteome has been described, it will be appreciated that where compounds effecting the interactions of specific proteins have already been identified, proteome data excluding the interactions of a specific protein or group of proteins could be input into the input store 10. The computer 2 would then be able to identify additional targets to complement the activity of the known compound or compounds. Thus in this way when a potential compound has been found to have some activity, complementary targets for therapy could then be identified.

Although the embodiments of the invention described with reference to the drawings comprise computer apparatus and processes performed in computer apparatus, the invention also extends to computer programs, particularly computer programs on or in a carrier, adapted for putting the invention into practice. The program may be in the form of source or object code or in any other form suitable for use in the implementation of the processes according to the invention. The carrier be any entity or device capable of carrying the program.

For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal which may be conveyed via electrical or optical cable or by radio or other means.

When a program is embodied in a signal which may be conveyed directly by a cable or other device or means, the carrier may be constituted by such cable or other device or means.

Alternatively, the carrier may be an integrated circuit in which the program is embedded, the integrated circuit being adapted for performing, or for use in the performance of, the relevant processes.

## Claims

1. A computer implemented method of identifying target proteins for targeting by drug therapies comprising:
obtaining (s2-1) proteome data for an organism to be targeted;
storing (s4-1) said proteome data as network data (40) within a computer (2), said network data (40) comprising data defining a plurality of nodes and a plurality of links between said nodes, wherein said nodes are associated with proteins and said links are indicative of protein interactions;
processing (s4-3) the stored network data (40) utilizing the computer (2) to determine for each node in the network defined by the stored network data (40) the number of links associated with each node;
ordering the nodes in the network defined by stored network data (40) on the basis of the number of links associated with a node;
identifying as hub nodes, a set of nodes in the ordered list associated with the greatest numbers of links; and
identifying as proteins for targeting by drug therapies, proteins associated with the identified hub nodes.

2. A method in accordance with claim 1, further comprising:
providing a database (32) identifying critical proteins for the activity of a host organism; and
identifying as proteins for targeting by drug therapies proteins associated with the identified hub nodes which do not correspond to proteins identified by data in the database (32) identifying critical proteins for the activity of a host organism.

3. A method in accordance with claim 1 or claim 2, further comprising:
providing a conservation database (30) identifying proteins in a host organism which are homologs of proteins in an organism to be targeted; and
identifying, as proteins for targeting by drug therapies, proteins associated with the identified hub nodes other than proteins identified by data in the conservation database (30).

4. A method in accordance with any of claims 1-3 further comprising:
obtaining (s2-1) proteome data for a host organism;
storing (s4-1) said proteome data as network data (40) within a computer (2), said network data (40) comprising data defining a plurality of nodes and a plurality of links between said nodes, wherein said nodes are associated with proteins and said links are indicative of protein interactions;
processing (s4-3) the stored network data for the host organism (40) utilizing the computer (2) to determine for each node in the network defined by the stored network data (40) the number of links associated with each node;
ordering the nodes in the network defined by stored network data for the host organism (40) on the basis of the number of links associated with a node;
identifying as hub nodes for the host organism, a set of nodes in the ordered list associated with the greatest numbers of link;
identifying as proteins for targeting by drug therapies, proteins associated with the identified hub nodes in the proteome of an organism to be targeted which are not proteins associated with the identified hub nodes in the proteome of the host organism.

5. A method of manufacturing a therapeutic drug comprising:
identifying one or more target proteins for targeting by drug therapies by performing a method (s2-2) in accordance with any preceding claim;
identifying (s2-6) one or more compounds which react with the indentified one or more target proteins; and
manufacturing a therapeutic drug containing compounds identified as reacting with the identified target proteins.

6. A computer readable medium storing computer interpretable instructions which when interpreted by a programmable computer cause the computer to perform a method in accordance with any of claims 1-4.

7. An information processing apparatus (2) for identifying target proteins for targeting by drug therapies comprising:
a data store (10) configured to store proteome data for an organism to be targeted as network data (40) defining a plurality of nodes and a plurality of links between said nodes wherein said nodes are associated with proteins and said links are indicative of protein interactions;
a processing unit (22) operable to process network data (40) stored in said data store (10) to determine for each of the nodes of the network defined by the stored network data (40), the number of links associated with each node; order the nodes in the network defined by stored network data (40) on the basis of the number of links associated with each node; and identify as hub nodes, a set of nodes in the ordered list associated with the greatest numbers of links; and
an output unit operable to output data identifying one or more target proteins for targeting by drug therapies proteins corresponding to the identified hub nodes.

8. An information processing apparatus (2) in accordance with claim 7, further comprising:
a database (32) storing data identifying critical proteins for the activity of a host;
wherein the output unit is operable to output as data identifying one or more target proteins, proteins associated with hub nodes identified by the processing unit (22) which do not correspond to proteins identified by data in the database (32).

9. An information processing apparatus (2) in accordance with claim 7, further comprising:
a conservation database (30) identifying proteins of a host organism which are homologs of proteins in an organism to be targeted;
wherein the output unit is operable to output as data identifying one or more target proteins, proteins associated with hub nodes identified by the processing unit (22) which are not proteins identified by data in the conservation database (30).

## Patentansprüche

1. Computerimplementiertes Verfahren zum Identifizieren von Zielproteinen zum gezielten Behandeln ("targeting") durch medikamentöse Therapieverfahren, das aufweist:
Erhalten (s2-1) proteomischer Daten für einen gezielt zu behandelnden Organismus;
Speichern (s4-1) der proteomischen Daten als Netzwerkdaten (40) in einem Computer (2), wobei die Netzwerkdaten (40) Daten aufweisen, die eine Vielzahl von Knoten und eine Vielzahl von Verknüpfungen zwischen den Knoten definieren, wobei die Knoten mit Proteinen assoziiert sind und die Verknüpfungen Protein-Wechselwirkungen anzeigen;
Verarbeiten (s4-3) der gespeicherten Netzwerkdaten (40) unter Verwendung des Computers (2), um für jeden Knoten in dem durch die gespeicherten Netzwerkdaten (40) definierten Netzwerk die Anzahl von Verknüpfungen zu bestimmen, die mit jedem Knoten assoziiert sind;
Sortieren der Knoten in dem durch die gespeicherten Netzwerkdaten (40) definierten Netzwerk auf Grundlage der Anzahl von Verknüpfungen, die mit einem Knoten assoziiert sind;
Identifizieren, als Zentralknoten, einen Satz von Knoten in der sortierten Liste, die mit den höchsten Anzahlen von Verknüpfungen assoziiert sind; und
Identifizieren von Proteinen, die mit den identifizierten Zentralknoten assoziiert sind, als Proteine zum gezielten Behandeln ("targeting") durch medikamentöse Therapieverfahren.

2. Verfahren in Übereinstimmung mit Anspruch 1, das weiterhin aufweist:
Bereitstellen eine Datenbank (32), die für die Aktivität eines Gastorganismus wichtige Proteine identifiziert; und
Identifizieren, als Proteine zum gezielten Behandeln ("targeting") durch medikamentöse Therapieverfahren, von Proteinen, die mit den identifizierten Zentralknoten assoziiert sind und die nicht Proteinen entsprechen, die durch Daten in der die für die Aktivität eines Gastorganismus wichtige Proteine identifizierenden Datenbank (32) identifiziert werden.

3. Verfahren in Übereinstimmung mit Anspruch 1 oder Anspruch 2, das weiterhin aufweist:
Bereitstellen einer Konservierungs-Datenbank (30), die Proteine in einem Gastorganismus identifiziert, die Homologe von Proteinen in einem gezielt zu behandelnden Organismus sind; und
Identifizieren, als Proteine zum gezielten Behandeln ("targeting") durch medikamentöse Therapieverfahren, von Proteinen, die mit den identifizierten Zentralknoten assoziiert und von den durch die Daten in der Konservierungs-Datenbank (30) identifizierten Proteinen verschieden sind.

4. Verfahren in Übereinstimmung mit einem der Ansprüche 1 bis 3, das weiterhin aufweist:
Erhalten (s2-1) proteomischer Daten für einen Gastorganismus;
Speichern (s4-1) der proteomischen Daten als Netzwerkdaten (40) in einem Computer (2), wobei die Netzwerkdaten (40) Daten aufweisen, die eine Vielzahl von Knoten und eine Vielzahl von Verknüpfungen zwischen den Knoten definieren, wobei die Knoten mit Proteinen assoziiert sind und die Verknüpfungen Protein-Wechselwirkungen anzeigen;
Verarbeiten (s4-3) der für den Gastorganismus gespeicherten Netzwerkdaten (40) unter Verwendung des Computers (2), um für jeden Knoten in dem durch die gespeicherten Netzwerkdaten (40) definierten Netzwerk die Anzahl von mit jedem Knoten assoziierten Verknüpfungen zu bestimmen;
Sortieren der Knoten in dem durch die für den Gastorganismus gespeicherten Netzwerkdaten (40) definierten Netzwerk auf Grundlage der Anzahl von mit einem Knoten assoziierten Verknüpfungen;
Identifizieren, als Zentralknoten für den Gastorganismus, einen Satz von Knoten in der sortierten Liste, die mit den höchsten Anzahlen von Verknüpfungen assoziiert sind;
Identifizieren, als Proteine zum gezielten Behandeln ("targeting") durch medikamentöse Therapieverfahren, Proteine, die mit den identifizierten Zentralknoten in dem Proteom eines gezielt zu behandelnden Organismus assoziiert und nicht Proteine sind, die mit den im Proteom des Gastorganismus identifizierten Zentralknoten assoziiert sind.

5. Verfahren zum Herstellen eines therapeutischen Medikaments, das aufweist:
Identifizieren eines oder mehrer Zielproteine zum gezielten Behandeln ("targeting") durch medikamentöse Therapieverfahren durch Ausführen eines Verfahrens (s2-2), das in Übereinstimmung mit einem der vorangegangenen Ansprüche ist;
Identifizieren (s2-6) eines oder mehrerer Komponenten, die mit den identifizierten einen oder mehreren Zielproteinen wechselwirken; und
Herstellen eines therapeutischen Medikaments, das Komponenten aufweist, die als mit den identifizierten Zielproteinen wechselwirkend identifiziert sind.

6. Computerlesbares Medium, das durch einen Computer auswertbare Anweisungen speichert, welche, wenn sie durch einen programmierbaren Computer ausgewertet werden, den Computer dazu veranlassen, ein Verfahren in Übereinstimmung mit einem der Ansprüche 1 bis 4 auszuführen.

7. Datenverarbeitende Vorrichtung (2) zum Identifizieren von Zielproteinen zum gezielten Behandeln ("targeting") durch medikamentöse Therapieverfahren, das aufweist:
einen Datenspeicher (10), ausgestaltet zum Speichern proteomischer Daten für einen gezielt zu behandelnden Organismus als Netzwerkdaten (40), die eine Vielzahl von Knoten und eine Vielzahl von Verknüpfungen zwischen den Knoten definiert, wobei die Knoten mit Proteinen assoziiert sind und die Verknüpfungen Protein-Wechselwirkung anzeigen;
eine Verarbeitungseinheit (22), betriebsfähig zum Verarbeiten von Netzwerkdaten (40), die in dem Datenspeicher (10) gespeichert sind, um für jeden der Knoten des durch die gespeicherten Netzwerkdaten (40) definierten Netzwerks die Anzahl von mit jedem Knoten assoziierten Verknüpfungen zu bestimmen; die Knoten in dem durch die gespeicherten Netzwerkdaten (40) definierten Netzwerk auf Grundlage der Anzahl von mit jedem Knoten assoziierten Verknüpfungen zu sortieren; und einen Satz von Knoten in der sortierten Liste, die mit den höchsten Anzahlen von Verknüpfungen assoziiert sind, als Zentralknoten zu identifizieren; und
eine Ausgabeeinheit, betriebsfähig zum Ausgeben von Daten, die ein oder mehr Zielproteine zum gezielten Behandeln ("targeting") durch medikamentöse Therapieverfahren identifizieren, wobei Proteine den identifizierten Zentralknoten entsprechen.

8. Datenverarbeitende Vorrichtung (2) in Übereinstimmung mit Anspruch 7, die weiterhin aufweist:
eine Datenbank (32), die für die Aktivität eines Gasts wichtige Proteine identifizierende Daten speichert;
wobei die Ausgabeeinheit betriebsfähig ist zum Ausgeben, als ein oder mehrere Zielproteine identifizierende Daten, von Proteinen, die mit durch die Verarbeitungseinheit (22) identifizierten Zentralknoten assoziiert sind und die nicht Proteinen entsprechen, die durch Daten in der Datenbank (32) identifiziert werden.

9. Datenverarbeitende Vorrichtung (2) in Übereinstimmung mit Anspruch 7, die weiterhin aufweist:
eine Konservierungs-Datenbank (30), die Proteine eines Gastorganismus identifiziert, die Homologe von Proteinen in einem gezielt zu behandelnden Organismus sind;
wobei die Ausgabeeinheit betriebsfähig ist zum Ausgeben, als ein oder mehrere Zielproteine identifizierende Daten, von Proteinen, die mit durch die Verarbeitungseinheit (22) identifizierten Zentralknoten assoziiert und nicht Proteine sind, die durch Daten in der Konservierungs-Datenbank (30) identifiziert werden.

## Revendications

1. Procédé mis en oeuvre par ordinateur pour l'identification de protéines cibles pour ciblage par des thérapies médicamenteuses comprenant les étapes consistant à :
obtenir (s2-1) des données de protéome pour un organisme à cibler;
stocker (s4-1) lesdites données de protéome sous la forme de données de réseau (40) dans un ordinateur (2), lesdites données de réseau (40) comprenant des données définissant une pluralité de noeuds et une pluralité de liaisons entre lesdits noeuds, lesdits noeuds étant associés à des protéines et lesdites liaisons étant indicatives d'interactions de protéines ;
traiter (s4-3) les données de réseau stockées (40) en utilisant l'ordinateur (2) pour déterminer, pour chaque noeud du réseau défini par les données de réseau stockées (40), le nombre de liaisons associées à chaque noeud ;
ordonner les noeuds au sein du réseau défini par les données de réseau stockées (40), sur la base du nombre de liaisons associées à un noeud ;
identifier en tant que noeuds pivots un jeu de noeuds dans la liste ordonnée associés au plus grand nombre de liaisons ; et
identifier en tant que protéines pour ciblage par des thérapies médicamenteuses, des protéines associées aux noeuds pivots identifiés.

2. Procédé selon la revendication 1, comprenant en outre :
la fourniture d'une base de données (32) identifiant des protéines critiques pour l'activité d'un organisme hôte ; et
l'identification en tant que protéines pour ciblage par des thérapies médicamenteuses, de protéines associées aux noeuds pivots identifiés qui ne correspondent pas aux protéines identifiées par des données dans la base de données (32) identifiant des protéines critiques pour l'activité d'un organisme hôte.

3. Procédé selon la revendication 1 ou la revendication 2, comprenant en outre :
la fourniture d'une base de données de conservation (30) identifiant des protéines dans un organisme hôte qui sont des homologues de protéines dans un organisme destiné à être ciblé ; et
l'identification, en tant que protéines pour ciblage par des thérapies médicamenteuses, de protéines associées aux noeuds pivots identifiés autres que des protéines identifiées par des données dans la base de données de conservation (30).

4. Procédé selon l'une quelconque des revendications 1 à 3, comprenant en outre les étapes consistant à :
obtenir (s2-1) des données de protéome pour un organisme hôte ;
stocker (s4-1) lesdites données de protéome sous la forme de données de réseau (40) dans un ordinateur (2), lesdites données de réseau (40) comprenant des données définissant une pluralité de noeuds et une pluralité de liaisons entre lesdits noeuds, lesdits noeuds étant associés à des protéines et lesdites liaisons étant indicatives d'interactions de protéines ;
traiter (s4-3) les données de réseau stockées pour l'organisme hôte (40) en utilisant l'ordinateur (2) pour déterminer, pour chaque noeud du réseau défini par les données de réseau stockées (40), le nombre de liaisons associées à chaque noeud ;
ordonner les noeuds au sein du réseau défini par les données de réseau stockées pour l'organisme hôte (40), sur la base du nombre de liaisons associées à un noeud ;
identifier en tant que noeuds pivots un jeu de noeuds dans la liste ordonnée associés au plus grand nombre de liaisons ; et
identifier comme protéines pour ciblage par des thérapies médicamenteuses, les protéines associées au noeuds pivots identifiés dans le protéome d'un organisme destiné à être ciblé qui ne sont pas des protéines associées au noeuds pivots identifiés dans le protéome de l'organisme hôte.

5. Un procédé de fabrication d'un médicament thérapeutique comprenant :
l'identification d'une ou plusieurs protéines cibles pour ciblage par des thérapies médicamenteuses par la mise en oeuvre d'un procédé (s2-2) selon l'une quelconque des revendications précédentes ;
l'identification (s2-6) d'un ou de plusieurs composés qui réagissent avec le ou les protéines cibles identifiées ; et
la fabrication d'un médicament qui contient des composés identifiés comme réagissant avec les protéines cibles identifiées.

6. Un support lisible par ordinateur stockant des instructions interprétables par ordinateur qui, lors de leur exécution par un ordinateur programmable, provoquent la réalisation du procédé selon l'une quelconque des revendications 1 à 4 par l'ordinateur.

7. Un dispositif de traitement de l'information (2) pour l'identification de protéines cibles pour ciblage par des thérapies médicamenteuses, comprenant :
une mémoire de données (10) configurée pour stocker des données de protéome pour un organisme destiné à être ciblé sous forme de données de réseau (40) définissant une pluralité de noeuds et une pluralité de liaisons entre lesdits noeuds dans lequel lesdits noeuds sont associés à des protéines et lesdits liaisons sont indicatives d'interactions de protéines ;
une unité de traitement (22) pouvant fonctionner pour traiter les données de réseau stockées (40) dans ladite mémoire de données (10) pour déterminer, pour chacun parmi les noeuds du réseau définis par les données de réseau stockées (40), le nombre de liaisons associées à chaque noeud ; pour ordonner les noeuds au sein du réseau défini par les données de réseau stockées (40) sur la base du nombre de liaisons associées à chaque noeud ; et pour identifier comme noeuds pivots, un jeu de noeuds dans la liste ordonnée associés aux plus grands nombres de liaisons ; et
une unité de sortie pouvant fonctionner pour sortir des données identifiant une ou plusieurs protéines cibles pour ciblage par des thérapies médicamenteuses, les protéines correspondant aux noeuds pivots identifiés.

8. Dispositif de traitement d'informations (2) selon la revendication 7, comprenant en outre :
une base de données (32) stockant des données identifiant des protéines critiques pour l'activité d'un hôte ;
dans lequel l'unité de sortie peut fonctionner afin de sortir en tant que données identifiant une ou plusieurs protéines cibles, les protéines associées aux noeud pivots identifiés par l'unité de traitement (22) qui ne correspond pas aux protéines identifiées par les données dans la base de données (32).

9. Dispositif de traitement de l'information (2) selon la revendication 7, comprenant en outre :
une base de données de conservation (30) identifiant les protéines d'un organisme hôte qui sont les homologues de protéines dans un organisme à cibler ;
dans lequel l'unité de sortie peut fonctionner afin de sortir en tant que données identifiant une ou plusieurs protéines cibles, les protéines associées aux noeuds pivots identifiés par l'unité de traitement (22) qui ne sont pas des protéines identifiées par les données dans la base de données de conservation (30).
